# EUROPEAN PATENT APPLICATION

(11) **EP 4 707 381 A1**
(43) Date of publication of application: **11.03.2026**
(21) Application number: 24797228.4
(22) Date of filing: 26.04.2024
(51) Int. Cl.: C12N 1/21, C12N 1/20, C12N 15/31, C12N 15/53, C12P 1/04, C12P 13/04, C12P 13/14, C12P 21/00

(54) **NITROGEN-FIXING BACTERIUM, AND METHOD FOR PRODUCING AMMONIA-, AMINO ACID- OR PROTEIN-CONTAINING CULTURE USING NITROGEN-FIXING BACTERIUM**

(30) Priority: 27.04.2023 JP 2023073543
(71) Applicant: Kikkoman Corporation, Noda-shi, Chiba 278-8601 (JP); The University of Tokyo, Bunkyo-ku, Tokyo 113-8654 (JP)
(72) Inventor: ITO,Yusuke, Noda-shi, Chiba 278-8601 (JP); YOSHIDOME, Daisuke, Tokyo 113-8654 (JP); HIDAKA, Makoto, Tokyo 113-8654 (JP); MATSUDA KOSONO, Saori, Tokyo 113-8654 (JP); NISHIYAMA, Makoto, Tokyo 113-8654 (JP)
(74) Representative: Gill Jennings & Every LLP
(86) International application number: PCT/JP2024/016609
(87) International publication number: WO 2024/225486

(57) **Abstract**

Nitrogen-fixing bacteria having a gene cassette that produces high expression of the known nifA gene transferred into the regulatory region of the nifA gene, have been found to shed the gene cassette after repeated subculturing in the absence of a selection agent such as kanamycin. A nifA gene expression cassette comprising the nifA gene, and tac or trc promoter, Lac operator and the ribosome binding site (RBS), disposed in a manner allowing expression of the nifA gene, has been transferred into a gene locus other than the nifL-nifA gene locus, to obtain nitrogen-fixing bacteria without such shedding of the gene cassette, even after subculturing. A method has been devised for producing amino acid- or protein-containing culture product or a meat alternative, using the nitrogen-fixing bacteria.

## Description

### FIELD

The present invention relates to nitrogen-fixing bacteria with enhanced nitrogen fixing ability, and to the use of the nitrogen-fixing bacteria. More specifically, the invention relates to a method for producing an amino acid- or protein-containing culture by culturing an ammonia-utilizing microorganism in medium prepared from an ammonia-accumulated culture that has been obtained by culturing nitrogen-fixing bacteria. The amino acid- or protein-containing culture produced in this manner can be used for purposes such as foods and feeds. Moreover, since the nitrogen-fixing bacteria of the invention have more enhanced nitrogen-fixing ability than conventional nitrogen-fixing bacteria, the bacteria themselves can be efficiently used as protein sources in foods and feeds. The nitrogen-fixing bacteria of the invention are also not limited to food products and may be used for production of microbial preparations or fertilizers that promote plant growth, or for production of ammonia for use as fuel.

### BACKGROUND

The most commonly used method for producing ammonia is currently the Haber-Bosch process. In this method, hydrogen molecules produced by steam reforming of fossil fuels (coal, petroleum and methane gas) are reacted with nitrogen molecules in the atmosphere to produce ammonia, but the method requires considerable energy and cost for operation. In light of problems such as fossil fuel depletion, expectations are high for production of ammonia which utilizes the nitrogen-fixing ability of microorganisms, and the use of ammonia as fertilizer or fuel. The ammonia utilization ability of microorganisms is also expected to be useful for conversion of the produced ammonia to proteins and amino acids, for use in foods and feeds.

The ability to convert nitrogen molecules in the atmosphere to ammonia is referred to as "nitrogen-fixing ability". Several hundred microorganisms with nitrogen-fixing ability have been discovered to date. All microorganisms with nitrogen-fixing ability are microorganisms in the Bacteria domain (Eubacteria) or Archaea domain (Archaebacteria), and herein these will be collectively referred to as "nitrogen-fixing bacteria". Nitrogen-fixing bacteria are able to grow with nitrogen molecules as their sole nitrogen source.

The enzymes involved in nitrogen-fixing ability are nitrogenases, which catalyze the following reaction under ideal reaction conditions *in vitro.*

N₂ + 8H⁺ + 8e⁻ + 16ATP → 2NH₃ + H₂ + 16ADP + 16Pi

Nitrogenase is a complex enzyme comprising dinitrogenase, which reduces nitrogen molecules, and dinitrogenase reductase which transfers electrons to dinitrogenase. Dinitrogenase has three subtypes, the Mo-type, V-type and Fe-type, the dinitrogenase type of all nitrogen-fixing bacteria being the Mo-type, which has an iron-molybdenum cofactor at the active center. Nitrogen-fixing bacteria therefore cannot exhibit nitrogen-fixing ability without a sufficient amount of molybdenum and iron present in the environment around them. Furthermore, if utilizable nitrogen sources (ammonium salts, nitrates and protein degradation products such as peptones) are present in their own surrounding environment, nitrogen-fixing bacteria do not exhibit nitrogen-fixing ability but rather proliferate by utilizing those nitrogen sources. In other words, nitrogen fixation by nitrogen-fixing bacteria takes place only when nitrogen sources have been depleted from their surrounding environment.

Nitrogen-fixing bacteria present in the natural world carry out nitrogen immobilization only in a very limited environment, and the amounts produced have been insufficient for industrial utilization. It has therefore been attempted to augment nitrogen-fixing ability by genetic modification of nitrogen-fixing bacteria. Modification of a repressor-activator system comprising nifL-nifA that regulates expression of the nitrogenase gene group has been successfully carried out (NPL 1: J. Bacteriol. (1999) 181(23) 7356-62, NPL 2: PLoS Genet. (2021) 17 (6)e1009617, NPL 3: Microb Cell Fact. (2020) 19(1):107, NPL 4: Applied Microbiology and Biotechnology (2922)106:5051-5061). It has also been reported that destruction of ammonia transporter genes, and suppression of nitrogen assimilation by glutamine synthase mutations, increase the amount of ammonia accumulating in culture medium (NPL 5: Appl Environ Microbiol (2015) 81(13):4316-28, NPL 6: Metab Eng. (2014) 23, 154-64).

It has also been attempted to produce plants and microorganic biomass from accumulated ammonia by co-culturing algae with bacterial strains that store ammonia in the medium (NPL 6, NPL 7: Metab Eng. 2017 Mar; 40:59-68.), but this has not been satisfactory for application to foods. On the other hand, nitrogen-fixing bacteria capable of ammonia accumulation, having gene modifications that regulate the nifL gene, have been reported to have unstable accumulation of ammonia or to be unstable when the cells are stored under frozen conditions (NPL 5). It has also been reported that the production of ammonia by nitrogen-fixing bacteria is still inadequate, and that it is difficult to achieve ammonia production of 30 mM (510 mg/L) or greater (NPL 3). Much room for improvement therefore still remains for production of ammonia by gene modification of nitrogen-fixing bacteria.

### [CITATION LIST]

### [NON PATENT LITERATURE]

[NPL 1] J. Bacteriol. (1999) 181 (23) 7356-62
[NPL 2] PLoS Genet. (2021) 17 (6)e1009617
[NPL 3] Microb Cell Fact. (2020) 19(1):107
[NPL 4] Applied Microbiology and Biotechnology (2922) 106:5051-5061
[NPL 5] Appl Environ Microbiol (2015) 81(13):4316-28
[NPL 6] Metab Eng. (2014) 23,154-64
[NPL 7] Metab Eng. (2017) 40:59-68.

### SUMMARY

### [TECHNICAL PROBLEM]

The present inventors have prepared nitrogen-fixing bacteria capable of accumulating ammonia, and have carried out culturing using nitrogen-fixing bacteria having a gene cassette with high expression of the nifA gene introduced into the control region of the conventionally known nifA gene, for the purpose of producing a culture containing ammonia, amino acids or proteins, and have found that the gene cassette is shed in the absence of a selection agent such as kanamycin.

### [SOLUTION TO PROBLEM]

The present inventors have conducted diligent research with the aim of obtaining nitrogen-fixing bacteria that do not exhibit impaired ammonia accumulation even with repeated culturing, and have completed this invention upon finding that it is possible to stably accumulate ammonia at high concentration by transferring a nifA gene expression cassette, comprising the nifA gene with an expression regulatory sequence and expression regulatory gene disposed in a manner allowing expression of the nifA gene, into a gene locus other than the nifL-nifA gene locus.

The present invention relates to the following:
[1] Nitrogen-fixing bacteria retaining the endogenous nifL-nifA gene locus, and having the nifA gene introduced by homologous recombination into a genomic region other than that of the nifL-nifA gene locus.
[2] Nitrogen-fixing bacteria according to [1] above, wherein a nifA gene expression cassette comprising the nifA gene and an expression regulatory sequence disposed in manner allowing regulation of expression of the nifA gene, is introduced by homologous recombination into a gene locus other than the nifL-nifA gene locus.
[3] Nitrogen-fixing bacteria according to [1] or [2] above, wherein the nitrogen-fixing bacteria are *Azotobacter* bacteria.
[4] Nitrogen-fixing bacteria according to any one of [1] to [3] above, wherein the nitrogen-fixing bacteria are *Azotobacter vinelandii* bacteria.
[5] Nitrogen-fixing bacteria according to any one of [1] to [4] above, wherein the nifA gene expression cassette is introduced at a gene locus other than the nifL-nifA gene locus using the double crossover method.
[6] An ammonia-accumulated culture product obtained by culturing nitrogen-fixing bacteria according to any one of [1] to [5] above in nitrogen limited medium in the absence of a selection agent.
[7] The culture product according to [7] above, which contains ammonia at 510 mg/L or greater.
[8] A method for producing an ammonia-accumulated culture product obtained by culturing nitrogen-fixing bacteria according to any one of [1] to [5] above in nitrogen limited medium in the absence of a selection agent.
[9] A method for producing an amino acid- or protein-containing culture product, the method comprising:
   a step of obtaining an ammonia-accumulated culture product by the method according to [8] above, and
   a step of culturing an ammonia-utilizing microorganism in the ammonia-accumulated culture product to obtain an amino acid- or protein-containing culture product.
[10] The method according to [9] above, which comprises a step of culturing nitrogen-fixing bacteria and inducing expression of the transferred nifA gene.
[11] The method according to [9] above, wherein the ammonia-accumulated culture product contains a culture supernatant and/or cell homogenate.
[12] The method according to [9] above, wherein the amino acid- or protein-containing culture product is used for a food.
[13] The method according to [9] above, wherein a sugar is added to the ammonia-accumulated culture product in which the ammonia-utilizing microorganism is to be cultured.
[14] The method according to [9] above, wherein the ammonia-utilizing microorganism is a microorganism belonging to *Corynebacterium,* a microorganism belonging to *Bacillus*, a microorganism belonging to *Saccharomyces,* a microorganism belonging to *Aspergillus,* or a microorganism belonging to *Fusarium.*

### [ADVANTAGEOUS EFFECTS OF INVENTION]

The nitrogen-fixing bacteria of the invention do not shed their transferred gene cassettes even when cultured in the absence of a selection agent. This allows culturing in the absence of a selection agent and enables stable production of ammonia. Any ammonia-utilizing microorganism may be cultured in medium prepared from an ammonia-accumulated culture product cultured in the absence of a selection agent. By using the nitrogen-fixing bacteria of the invention it is possible to produce ammonia at higher volume than with conventional nitrogen-fixing bacteria. The produced ammonia can therefore be used for highly efficient production of ammonia-utilizing microorganism biomass, as well as substance production. Moreover, since the nitrogen-fixing ability is enhanced over conventional nitrogen-fixing bacteria, the cells themselves can be more efficiently used as protein sources for foods and feeds. In addition, not only can the highly produced ammonia be efficiently utilized as fertilizer or fuel, but the bacteria may also be added to crop fields and applied as a microbial preparation to further reduce amounts of nitrogen fertilizer required.

### BRIEF DESCRIPTION OF DRAWINGS

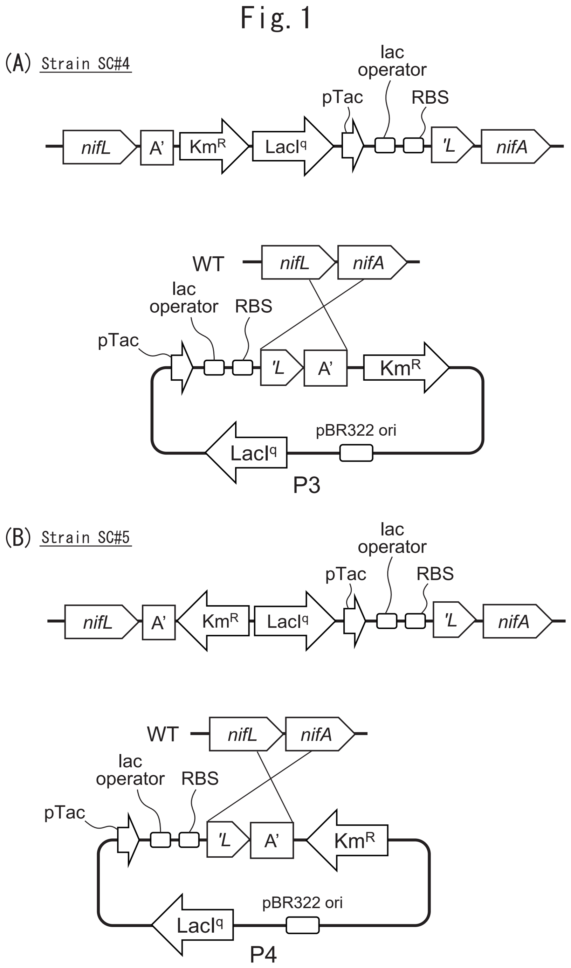

Fig. 1 schematically shows a gene expression cassette transferred into the nifL-nifA gene locus. Shown are schematic diagrams for strain SC#4 (A) having transferred into the gene locus a gene expression cassette with Ptac and Km oriented in the same orientation, and strain SC#5 (B) having transferred into the gene locus a gene expression cassette with Ptac and Km in opposite orientations, the depiction showing the nifL-nifA gene locus after transfer of the gene expression cassette, and introduction of the gene expression cassette into the nifL-nifA gene locus by homologous recombination from a plasmid containing the gene expression cassette.
Fig. 2 shows the design of primers for detection of gene expression cassettes for the wild type (WT), SC#4 and SC#5 (A), and the results of PCR amplification of nifL-nifA gene loci provided for preculturing and main culturing of SC#4 and SC#5 ((B) after preculturing, (C) after main culturing).
Fig. 3 schematically shows a nifA gene expression cassette introduced into the algU gene locus. The nifA gene expression cassette includes the nifA gene and the tac or trc promoter, lacI gene and ribosome binding site (RBS), disposed in a manner allowing expression of the nifA gene. A strain having the nifA gene expression cassette introduced into the algU gene locus was designated as the nifA high-expressing strain (DC#3).
Fig. 4 shows the OD600 value (A), pH (B), ARA content (C) and ammonia concentration (D), with or without addition of 0.3 mM IPTG during culturing of DC#3 in Burk's medium. As a control, the wild type (WT) was cultured in Burk's medium.
Fig. 5 shows the design of primers for detection of gene expression cassettes for the wild type (WT) and DC#3 (A), and the results of PCR amplification of nifL-nifA gene loci provided for preculturing and main culturing of DC#3 ((B) after preculturing, (C) after main culturing).
Fig. 6 shows the results of proliferation of *Bacillus subtilis* (A), *Saccharomyces cerevisiae* (B) and *Corynebacterium glutamicum* (C) after addition of sugar to the culture product obtained from culturing of DC#3.
Fig. 7 shows the results of glutamic acid production by *Corynebacterium glutamicum,* with addition of sugars to culture product obtaining after culturing DC#3.

### DESCRIPTION OF EMBODIMENTS

One aspect of the invention relates to nitrogen-fixing bacteria having a nifA gene expression cassette introduced by homologous recombination into a gene locus other than the nifL-nifA gene locus. Another aspect of the invention relates to use of the nitrogen-fixing bacteria. Specifically, it relates to culturing nitrogen-fixing bacteria of the invention to cause high production of ammonia. The invention further relates to a method for efficiently producing an amino acid- or protein-containing culture product by culturing an ammonia-utilizing microorganism.

### [Nitrogen-fixing bacteria]

According to the invention, the nitrogen-fixing bacteria may be any bacteria wherein transcription of a nitrogen-fixing associated gene group is controlled by the nifL-nifA gene, and examples include microorganisms of the genus *Azotobacter,* and especially *Azotobacter vinelandii, Azotobacter chroococcum, Azotobacter beijerinckii, Azotobacter armeniacus, Azotobacter nigricans, Azotobacter paspali* and *Azotobacter salinestris.* A nifA gene expression cassette is introduced into a gene locus other than the endogenous nifL-nifA gene locus of the nitrogen-fixing bacteria. It is thus possible to obtain nitrogen-fixing bacteria retaining the endogenous nifL-nifA gene locus, and having the nifA gene introduced by homologous recombination into a genomic region other than the nifL-nifA gene locus. The transfer region other than the nifL-nifA gene locus may be selected in consideration of avoiding effects on nitrogen fixing activity even when disrupted, and as a region not essential for growth, although it may be a region coding for a gene, or any other region. The gene locus is preferably a region coding for a pseudogene, examples of which include algU (Avin_13660), Avin_ 03890, Avin_04200 and Avin_04280. The nifL-nifA gene locus is the gene locus coding for the nif gene group repressor nifL and activator nifA, which are adjacently situated. The gene nifA contributes to enhanced expression of the nitrogenase gene group, with enhanced nifA expression promoting expression of the nitrogenase gene group and thereby increasing nitrogen fixing activity and ammonia production. The nifL gene, on the other hand, interacts with nifA and suppresses nifA activity. Suppression of nifL expression and suppression of nifL function can therefore also contribute to ammonia production.

The term "gene expression cassette introduced into the nitrogen-fixing bacteria of the invention" is nucleic acid introduced in order to promote expression of the nitrogenase gene. The gene expression cassette may include one or more selected from the group consisting of the nifA gene, the expression regulatory sequence that regulates transcription of the nifA gene, and a drug resistance gene. Transferred sequences for high expression of the nifA gene in the prior art do not include the full length of the nifA gene, but a transcription regulating sequence and drug resistance gene can be incorporated in an expressible manner upstream from the nifA gene in the genome to allow regulation of nifA expression. The nifA gene expression cassette used for the invention, on the other hand, contains a gene expression cassette with the full length of the nifA gene as the transgene. Examples of the nifA gene expression cassette therefore include a nifA gene expression cassette comprising the nifA gene, and an expression regulatory sequence, such as a tac or trc promoter and Lac operator, as well as a ribosome binding site (RBS) and LacI, disposed in a manner allowing regulation of expression of the nifA gene. The nifA gene in the nifA gene expression cassette may be the host-derived nifA gene or a heterogenous nifA gene. These nifA genes may also have additional mutations that enhance nifA protein activity. Mutations that enhance nifA activity include mutations in regions where interaction with the repressor nifL takes place.

As an example, the nifA gene expression cassette may be introduced by homologous recombination, using the single crossover method or double crossover method, for example. When the nifA gene expression cassette has been introduced by homologous recombination using the single crossover method, the homologous site remains near the transferred gene cassette, and consequently repeated subculturing or long-term culturing may result in further homologous recombination and shedding of the gene cassette. A strain that has shed the gene cassette is superior to a strain that comprises the gene cassette, from the viewpoint of its growth properties. A strain that has shed the gene cassette is restored to the wild type, which no longer carries out nitrogen fixation when ammonia is present in the culture solution, but instead assimilates the accumulated ammonia for growth. In other words, the appearance of a strain that has shed the gene cassette causes ammonia accumulation to be reduced. When culturing is carried out on the laboratory level, a selection agent is normally added to the medium, with the selection pressure being constantly applied so as to avoid the problem of shedding of the gene cassette. However, it is preferred to avoid the use of selection agents when the purpose is utilization in food production or microbial preparation, or when other bacterial strains are also to be cultured, and culturing in the absence of a selection agent results in shedding of gene cassettes. It is therefore preferred to carry out the transfer by the double crossover method from the viewpoint of inhibiting shedding of the nifA gene expression cassette.

An expression regulatory sequence is any sequence that is capable of regulating gene expression. The expression regulatory sequence used may be any regulatory sequence that is well known in the technical field, examples of which include one or more selected from the group consisting of promoters, operators, ribosome binding sites and repressor genes. The promoter used may be any sequence that functions in nitrogen-fixing bacteria, and it may be a sequence originally conserved by such bacteria. There may also be used T7, pTac, pTrc, pBAD or pLac, for example, which are used for heterogenous expression in bacteria. Operators include operators that regulate transcription of lactose operon or arabinose operon in *Escherichia coli.* The presence of an operator allows gene expression to be turned on or off by administration of a substance that activates the operator. When using Lac operator, as one example, addition of the allolactose analog isopropyl-β-thiogalactopyranoside (IPTG) allows regulation of expression of a gene further downstream. The ribosome binding site (RBS) used may be a functional sequence wherein the ribosome of the nitrogen-fixing bacteria binds with the transcribed mRNA. The RBS is not particularly restricted so long as it is a sequence that functions in nitrogen-fixing bacteria. Repressor genes include lacI and araC. Linking the expression regulatory sequence in a manner allowing it to activate a transgene or a desired gene in a genome will promote expression of the transgene or desired gene. The expression regulatory sequence used in the gene locus to be transferred may also be utilized for transfer into the gene locus of nitrogen-fixing bacteria.

The drug resistance gene is a gene that can impart resistance against a specific selection agent such as an antibiotic. The drug resistance gene may therefore be referred to as an "antibiotic resistance gene". Any drug resistance gene known in the technical field may be used, but since appropriate drugs will differ depending on the type of bacteria, suitable drug resistance genes selected among those that function for each bacterium may be used. Examples include the kanamycin resistance gene, ampicillin resistance gene, penicillin resistance gene, chloramphenicol resistance gene and tetracycline resistance gene. By transferring a drug resistance gene and culturing in medium containing the corresponding drug, it is possible to facilitate selection of homologously recombined bacterial strains, while culturing those bacterial strains in medium containing the corresponding drug can also prevent shedding of the gene cassette. From the viewpoint of utilization in food production or microbial preparation, however, it is preferred to avoid using a drug. The use of such a drug is preferably avoided because any residual drug will interfere with growth when culturing other bacteria in medium prepared from the produced ammonia-accumulated culture product. It is therefore undesirable to use a drug during the culturing after strain selection. The nitrogen-fixing bacteria of the invention may also have the drug resistance gene removed after selection.

A transgene is a gene transferred into a specific bacterium. The transgene will usually be disposed in a manner allowing activation of a regulatory region. When a Lac-tac or trc system is used in a regulatory region, an operator regulating substance such as IPTG may be introduced to control expression of the transgene. The transgene may be any gene, but the nifA gene may be selected from the viewpoint of augmenting expression of the nifA gene. A partial sequence of the nifL gene ('L) may also be introduced upstream from the nifA gene. Gene expression can be regulated by introducing the regulatory region in a manner so that it is capable of activating a specific gene in the genome, without transgene transfer.

By transfer of the nifA gene expression cassette of the invention, the nitrogen-fixing bacteria of the invention will have enhanced nitrogen-fixing ability, and culturing in medium with limited nitrogen (hereunder referred to as "nitrogen limited medium") will cause nitrogen in the atmosphere to be fixed as ammonia, allowing it to accumulate in the cells or medium. Such cells or culture medium in which ammonia has accumulated will be referred to herein as "ammonia-accumulated culture product". Another aspect of the invention, therefore, relates to a method for producing an ammonia-accumulated culture product which comprises culturing nitrogen-fixing bacteria of the invention. The method for producing an ammonia-accumulated culture product comprises a step of inducing expression of the nifA gene when a predetermined cell concentration has been reached, whereby expression of the nifA gene results in accumulation of ammonia. Since production of ammonia in the medium increases the medium pH, the pH can be used as an index to judge production of ammonia. A centrifugation step and/or a filter sterilization step may be carried out to remove the cells and recover the medium alone to obtain an ammonia-accumulated culture product, or the cells may be included as well, or disrupted to obtain an ammonia-accumulated culture product. The ammonia-accumulated culture product contains ammonia in an amount of at least 10 mM (170 mg/L) or greater, more preferably 15 mM (340 mg/L) or greater and even more preferably 20 mM (510 mg/L) or greater. Depending on the culturing conditions, the ammonia-accumulated culture product may contain ammonia at 30 mM (680 mg/L) or greater and even more preferably 60 mM (1 g/L) or greater. The method for producing an ammonia-accumulated culture product according to the invention may also be referred to herein simply as "ammonia production method".

The medium used for culturing of the nitrogen-fixing bacteria of the invention may be any medium in which the nitrogen-fixing bacteria grow. It may be a nitrogen-free medium lacking a nitrogen source, or it may be a nitrogen source-limited medium with a limited amount of nitrogen source. The medium used for culturing of the nitrogen-fixing bacteria of the invention may include salts, carbon sources, pH adjustors and trace elements. These components may be appropriately selected according to the nutritional auxotrophy and ammonia accumulation properties of the bacteria. The pH of the nitrogen limited medium before culturing may be selected as desired in a range that does not inhibit proliferation of the nitrogen-fixing bacteria, with adjustment being made in the range of pH 5.5 to 10, for example, but the pH will increase as ammonia accumulates during culturing. The pH may therefore be controlled so that it remains constant. One example of medium to be used is Burk's medium which contains no nitrogen source. The types and amounts of salts, carbon sources and trace elements may be adjusted as appropriate to produce the pH and conditions for growth and nitrogen fixing activity.

Salts include salts composed of cations such as potassium, iron, sodium, magnesium and calcium ion, and anions such as phosphate ion, manganate ion, sulfate ion and chloride ion. The salts used may be appropriately selected according to the nutritional auxotrophy of the nitrogen-fixing bacteria. Examples include potassium monohydrogen phosphate, potassium dihydrogen phosphate, iron sulfate, sodium manganate, magnesium sulfate and calcium chloride, which salts can serve as suppliers for essential elements such as phosphorus, manganese, iron, potassium, magnesium and sulfur. Other salts may also be used for supply of essential elements. Of these salts, potassium dihydrogen phosphate and dipotassium hydrogen phosphate may be used as buffering agents.

Saccharides are used as carbon sources, examples of which include monosaccharides, disaccharides, sugar alcohols, polysaccharides, organic acids and alcohols. Such a saccharide may be glucose, mannose, galactose, fructose, xylose, sucrose, maltose, cellobiose, trehalose, mannitol, inositol, sorbitol or a starch hydrolysate, but usually glucose or sucrose is used. An organic acid may be used instead of a saccharide so long as bacteria are able to utilize it. Examples of organic acids for use as carbon sources include acetic acid and lactic acid, as well as organic acids in the TCA cycle such as oxaloacetic acid, citric acid, isocitric acid, 2-oxoglutaric acid, succinic acid, fumaric acid and malic acid. The concentration of the carbon source may be set to 1 g/L to 100 g/L in terms of glucose, and from the viewpoint of promoting growth the concentration is preferably 2 g/L or greater and more preferably 5 g/L or greater, and preferably 80 g/L or lower and more preferably 60 g/L or lower. A carbon source may also be added during the culturing.

### [Method for producing ammonia-accumulated culture product or amino acid- or protein-containing culture product]

The nitrogen-fixing bacteria of the invention are used in a method for producing an ammonia-accumulated culture product by immobilizing nitrogen in the atmosphere of the culture medium. Another aspect of the invention relates to a method for producing an amino acid- or protein-containing culture product using an ammonia-accumulated culture product as the starting material. The method for producing an amino acid- or protein-containing culture product comprises:
a step of culturing the nitrogen-fixing bacteria of the invention in nitrogen limited medium in the absence of a selection agent corresponding to a drug resistance gene, to obtain an ammonia-accumulated culture product, and
a step of culturing the ammonia-utilizing microorganism in medium prepared from the ammonia-accumulated culture product, to obtain an amino acid- or protein-containing culture product. With the nitrogen-fixing bacteria of the invention it is possible to produce ammonia-accumulated culture products without leading to shedding of transferred gene cassettes, even when culturing has been in the absence of a selection agent. This allows amino acid- or protein-containing culture products of food-level quality to be obtained in subsequent steps, without interfering with culturing of the ammonia-utilizing microorganisms.

The step of obtaining the ammonia-accumulated culture product may be carried out according to the method described above for producing an ammonia-accumulated culture product. Culture medium is then further prepared from the prepared ammonia-accumulated culture product. As an example, the ammonia-accumulated culture product may be supplied for centrifugation and sterile filter treatment, and the cells and solid portion removed to prepare a culture medium, or alternatively the culture medium may also contain the solid portion including cells or cell homogenate from the ammonia-accumulated culture product. Since such media contain ammonia but have the carbon source or trace elements consumed from the initial nitrogen limited medium, the necessary components may be supplemented depending on the type of ammonia-utilizing microorganism to be subsequently cultured. At minimum, saccharides such as glucose or sucrose may be added. According to yet another aspect, an ammonia-utilizing microorganism may be co-cultured in medium containing live cells of nitrogen-fixing bacteria, as the ammonia-accumulated culture product.

The ammonia-utilizing microorganism may be any bacteria which is a bacterium that utilizes ammonia in the aforementioned medium. Microorganisms suitable for production of amino acid- or protein-containing culture products for foods are preferred, and examples include *Corynebacterium* microorganisms, *Escherichia* microorganisms, *Enterobacter* microorganisms, *Pantoea* microorganisms, *Bacillus* microorganisms, *Saccharomyces* microorganisms, *Candida* microorganisms, *Aspergillus* microorganisms, *Fusarium* microorganisms, *Rhizopus* microorganisms, *Lactobacillus* microorganisms and *Pediococcus* microorganisms. Ammonia-utilizing microorganisms can be applied for production of various food products. The present invention also relates to a method for producing a meat alternative, comprising a step of processing an amino acid- or protein-containing culture product obtained by culturing the aforementioned microorganisms into edible meat (a meat alternative), or it may relate to a method for producing a fermented food comprising a step of processing an amino acid- or protein-containing culture product as a nitrogen source. For example, after having grown an *Aspergillus* microorganism, *Fusarium* microorganism or *Rhizopus* microorganism, which are types of filamentous fungi, the hyphae may be collected and used as starting material for a meat alternative. When the filamentous fungi hyphae are collected and shaped it is possible to produce the same degree of chewiness as muscular fibers. A meat alternative can be produced by mixing and shaping the hyphae with fats or oils, and further adding flavors. Fermented foods can also be produced by mixing biomass or fermentation starting materials of ammonia-utilizing microorganisms grown using ammonia-accumulated culture products. By substituting all or a portion of the ammonia-utilizing microorganic cells as a protein source instead of soybean, which is a starting material for koji, the method can also be applied for production of fermented foods such as soy sauce and miso. Examples of ammonia-utilizing microorganisms that may be used include *Saccharomyces* microorganisms and *Aspergillus* microorganisms, which allow production of miso and soy sauce as fermented foods.

All of the publications mentioned throughout the present specification are incorporated herein in their entirety by reference.

The Examples of the invention described below are intended to serve merely as illustration and do not limit the technical scope of the invention. The technical scope of the invention is limited solely by the description in the Claims. Modifications of the invention, such as additions, deletions or substitutions to the constituent features of the invention, are possible so long as the gist of the invention is maintained.

### [Example 1]

### Example 1: Preparation of strains with enhanced nifA expression

### (Construction of plasmid containing high expression cassette)

A plasmid containing a high expression cassette was constructed by bonding the cassette fragments using Gibson Assembly. Table 1 shows the sequences of the primers used for amplification of each fragment. The design was such as to provide sequences of 15 bp or longer at both ends of each primer homologous to the adjacent fragments. The primers used and the constructed plasmids are shown in Table 2. A 10 µL portion of Gibson Assembly reaction mixture was used to transform *E. coli* DH5α, and a drug selection marker in the plasmids was used to screen the colonies. The plasmids were extracted from the appearing colonies and subjected to sequence analysis to confirm that the plasmids were as designed (Fig. 1).

The target strains were then screened by transfer and homologous recombination of plasmids created in *Azotobacter vinelandii* DJ. First, *A. vinelandii* was cultured at 30°C in molybdenum-free Burk's+N agar medium (B-Mo medium), and subcultured twice. Next, shake culturing was carried out overnight at 30°C, 280 rpm in 5 mL of Burk's+N medium (B-Mo-Fe medium) containing no molybdenum or iron. Upon confirming that the cell solution had turned green, 200 µL of cell solution, 200 µL of Transformation Buffer (20 mM MOPS, 20 mM MgCl₂, pH 7.2) and 1 to 5 µg of DNA were suspended and incubated at ordinary temperature for 2 hours. After adding to 5 mL of Burk's+N medium (20 g/L sucrose, 0.6 g/L MgSO₄·7H₂O, 0.09 g/L CaCl₂·2H₂O, 1.5 g/L NH₄(CH₃CO₂), 0.036 mM FeSO₄·7H₂O, 5 µM Na₂ MoO₄·2H₂O, 0.8 g/L K₂HPO₄, 0.2 g/L KH₂PO₄) in a test tube, shake culturing was carried out overnight at 30°C, 280 rpm. Transformants were inoculated to Burk's+N agar medium containing 1 µg/mL kanamycin, and culturing was continued at 30°C. The obtained colonies were used in colony PCR to obtain strain SC#4 and strain SC#5 (Fig. 1).

**[Table 1]**

| Table 1. Primers used for plasmid construction | | |
|---|---|---|
| Primer name | Sequence | Sequence No. |
| F1 | gacgagttcttctgaAAGGATCTAGGTGAAGATC | 1 |
| R1 | agccttcgcgcatGCTGTTTCCTGTGTGAAATTG | 2 |
| F2 | aggaaacagcatgCGCGAAGGCTGCCGGATC | 3 |
| R2 | agtgagggttaattTCAGTTCAGGCGCACGAAG | 4 |
| F3 | cgtgcgcctgaactgaAATTAACCCTCACTAAAG | 5 |
| R3 | ttcacctagatccttTCAGAAGAACTCGTCAAG | 6 |
| F4 | AAGGATCTAGGTGAAGATCC | 7 |
| R4 | TCAGTTCAGGCGCACGAAG | 8 |
| F5 | gtgcgcctgaactgATCAGAAGAACTCGTCAAG | 9 |
| R5 | ttcacctagatccttCTACCGGGTAGGGGAGGC | 10 |
| F6 | TAATCATCGGCTCGTATAATGTGTG | 11 |
| R6 | ACGAGCCGATGATTAATTGTCAACAGCTCATTTC | 12 |

| | | |
|---|---|---|
| (Lower case letters indicate sequences homologous with adjacent fragment) | | |

**[Table 2]**

| Table 2. Primers and plasmids used for preparation of bacterial strains | | | | | | |
|---|---|---|---|---|---|---|
| Constructed plasmid | Fragment 1 | | Fragment 2 | | Fragment 3 | |
| | Template | Primer | Template | Primer | Template | Primer |
| P1 | pTrc-His2-TOPO/lacZ (Thermo Fisher) | F1, R1 | Strain DJ genomic DNA | F2, R2 | FRT-PGK-gb2-neo-FRT (Gene Bridges) | F3, R3 |
| P2 | P1 | F4, R4 | FRT-PGK-gb2-neo-FRT (Gene Bridges) | F5, R5 | | |
| P3 (used for preparation of SC#4) | P1 | F6, R6 | | | | |
| P4 (used for preparation of SC#5) | P2 | F6, R6 | | | | |

### Example 2: Culturing of nifA enhanced expression strain and confirmation of gene cassette

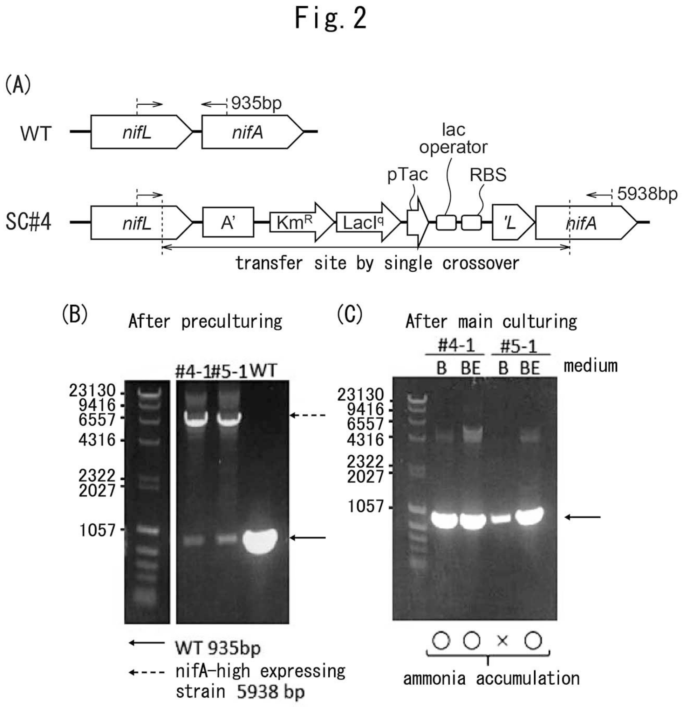

Strains SC#4 and 5 constructed as described in Example 1 were used to confirm that accumulation of ammonia had occurred. Preculturing (30°C, 280 rpm) of DJ wild type, SC#4 and SC#5 cells was carried out in a test tube containing 3 mL of Burk's+N medium with addition of kanamycin to a final concentration of 1 µg/mL. Next, each strain was was inoculated into 3 mL of Burk's medium (20 g/L sucrose, 0.6 g/L MgSO₄·7H₂O, 0.09 g/L CaCl₂-2H₂O, 0.036 mM FeSO₄·7H₂O, 5 µM Na₂ MoO₄·2H₂O, 0.8 g/L K₂HPO₄, 0.2 g/L KH₂PO₄) in the test tube to OD₆₀₀ = 0.04, and IPTG was added to a final concentration of 0. 03 mM for shake culturing (30°C, 280 rpm). The cell solution was collected at a time point of 72 hours or more after culturing, and was centrifuged at 15,000 rpm for 5 minutes. Ammonia accumulation was judged using the pH increase in the obtained culture supernatant as the index. The ammonia concentration in the supernatant was quantified using an F-kit (J.K. International) for each test group in which pH increase was observed. The results are shown in Table 3. The results showed test groups with and without increased pH compared to the culture solution of the wild type (WT). Measurement of the ammonia concentration in the test group with increased pH revealed that ammonia had accumulated at about 170 to 227 mg/L, but the phenotype was unstable and test groups without increase in pH were present. PCR was conducted by amplifying a high expression cassette using cell solutions of strains SC#4 and SC#5 before and after main culturing. The primers used are shown in Table 4. As a result it was found that the high expression cassette (5938 bp) present before the main culturing reverts to the wild type cassette (935 bp) during culturing (Fig. 2). This was attributed to the fact that strains in which transfer takes place by single crossover undergo recombination in a manner retaining the homologous site in proximity, so that recombination occurs again with the homologous site as a foothold, restoring the mutation to the cassette of the wild type.

**[Table 3]**

| Table 3. pH and ammonia concentrations of culture solutions at 72 hours from start of culturing | | | |
|---|---|---|---|
| Strain | Lot 1 | Lot 2 | Lot 3 |
| WT | 7.0 | 7.0 | 7.2 |
| SC#4 | 7.8 (170mg/L) | 7.8 (182mg/L) | 7.0 |
| SC#5 | 8.4 (227 mg/L) | 6.6 | 7.0 |

| | | | |
|---|---|---|---|
| Values in parentheses indicate ammonia concentrations. | | | |

**[Table 4]**

| Table 4. Primers used to confirm transferred fragment | | |
|---|---|---|
| Primer name | Sequence | Sequence No. |
| F7 | ATGGTGCACGTAGACACCGACTATC | 13 |
| R7 | AGAGTTCGGACTCGAGCAGGGTTTC | 14 |

### Example 3: Preparation of nifA high-expressing strain

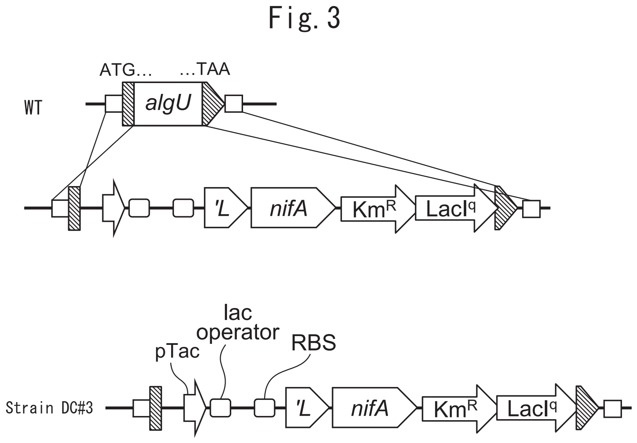

Since transfer of the gene cassette by single crossover was unstable, it was attempted to transfer into the algU gene locus by double crossover. A gene cassette for transfer was constructed. The fragments composing the cassette were bonded with Gibson Assembly (Fig. 3). Table 5 shows the sequences of the primers used for amplification of each fragment. The design was such as to provide sequences of approximately 15 bp at both ends of each primer, homologous to the adjacent fragments. The primers used and the constructed plasmids are shown in Table 6. The fragment for transfer into the algU gene locus was then amplified by PCR.

The target strains were screened by transfer and homologous recombination of plasmids created in *Azotobacter vinelandii* DJ. First, *A. vinelandii* was cultured at 30°C in molybdenum-free Burk's+N agar medium (B-Mo medium), and subcultured twice. Next, shake culturing was carried out overnight at 30°C, 280 rpm in 5 mL of Burk's+N medium (B-Mo-Fe medium) containing no molybdenum or iron. Upon confirming that the cell solution had turned green, 200 µL of cell solution, 200 µL of Transformation Buffer and 1 to 5 µg of DNA were suspended and the mixture was incubated at ordinary temperature for 2 hours. After adding to 5 mL of Burk's+N medium in a test tube, shake culturing was carried out overnight at 30°C, 280 rpm. Transformants were inoculated to Burk's+N agar medium containing 1 µg/mL kanamycin, and culturing was continued at 30°C. The obtained colonies were used in colony PCR to obtain strain DC#3.

**[Table 5]**

| Table 5. Primers used for plasmid construction (lower case letters indicate sequences homologous with adjacent fragment) | | |
|---|---|---|
| Primer name | Sequence | Sequence No. |
| F8 | GCTTATCGATGATAAGCTG | 15 |
| R8 | AGCTGTTTCCTGTGTGAAATTG | 16 |
| F9 | ttatcatcgataagcTCGCGGGTGAAAGGAACAC | 17 |
| R9 | acacaggaaacagctCCTTGGAAGAAGGCTCCTG | 18 |
| F10 | CCTTCATCAAAGCCTATCGTG | 19 |
| R10 | TCAGATCTTGCGCATGTGGATG | 20 |
| F11 | tgcgggaggtggatcAATTAACCCTCACTAAAGG | 21 |
| R11 | acagcttgtctgtaagTCAGAAGAACTCGTCAAG | 22 |
| F12 | gacgagttcttctgaCTTACAGACAAGCTGTGAC | 23 |
| R12 | aggctttgatgaaggTCACTGCCCGCTTTCCAGTC | 24 |
| F13 | AATTAACCCTCACTAAAG | 25 |
| R13 | TCAGATCTTGCGCATGTGGATG | 26 |
| F14 | tgcgggaggtggatcGTTTGACAGCTTATCATCG | 27 |
| R14 | ccggcagccttcgcgCATGCTGTTTCCTGTGTG | 28 |
| F15 | acaggaaacagcatgCGCGAAGGCTGCCGGATCG | 29 |
| R15 | tagtgagggttaattTCAGATCTTGCGCATGTGGATG | 30 |

**[Table 6]**

| Table 6. Primers and plasmids used for preparation of bacterial strains | | | | | | |
|---|---|---|---|---|---|---|
| Constructed plasmid | Fragment 1 | | Fragment 2 | | Fragment 3 | |
| | Template | Primer | Template | Primer | Template | Primer |
| P5 | pSTV28 (Takara Bio) | F8, R8 | Strain DJ genomic DNA | F9, R9 | FRT-PGK-gb2-neo-FRT (Gene Bridges) | F3, R3 |
| P6 | P5 | F10, R10 | FRT-PGK-gb2-neo-FRT (Gene Bridges) | F11, R11 | pTrc-His2-TOPO/lacZ (Thermo Fisher) | F12, R12 |
| P7 (used for preparation of DC#3) | P6 | F13, R13 | P3 | F14, R14 | Strain DJ genomic DNA | F15, R15 |

### Example 4: Culturing of DC#3 and confirmation of gene cassette retention in genome

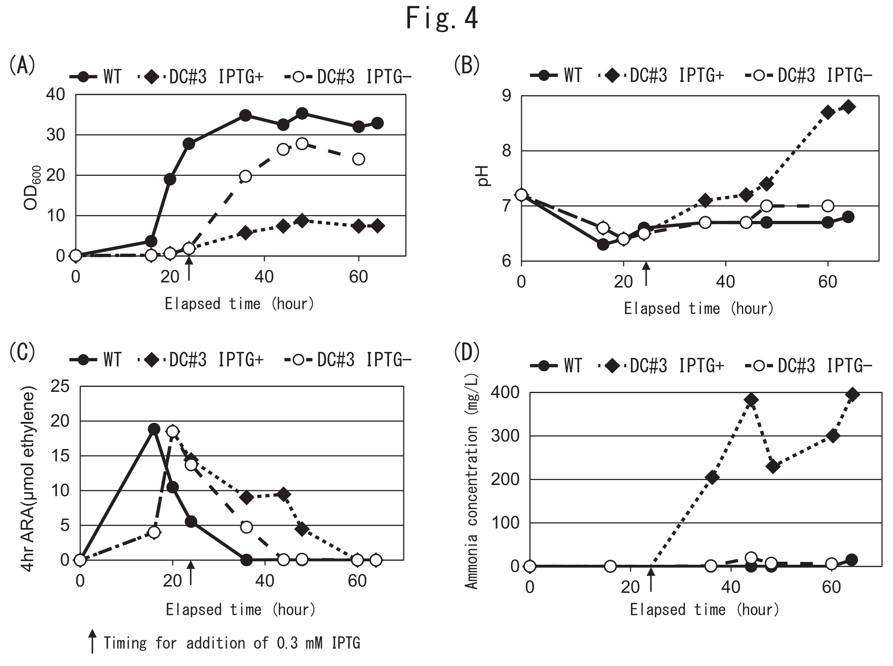

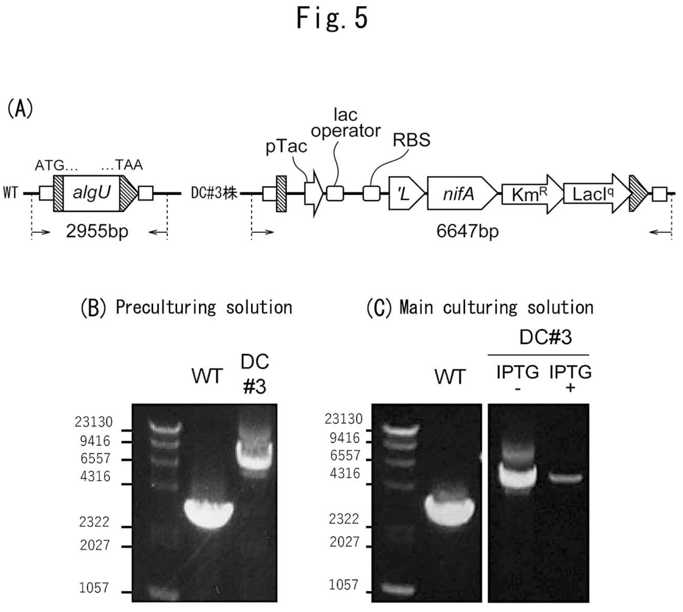

Strain DC#3 was used to confirm whether or not accumulation of ammonia occurred. Preculturing (30°C, 280 rpm) of DJ wild type and DC#3 cells was carried out in a test tube containing 5 mL of Burk's+N medium with addition of kanamycin to a final concentration of 1 µg/mL. It was then inoculated into a test tube containing 5 mL of Burk's medium to an OD₆₀₀ = 0.02, and shake culturing was carried out (30°C, 280 rpm). IPTG was added to a final concentration of 0. 3 mM 24 hours after the start of culturing. After elapse of each time period, the culture solution was collected and the OD₆₀₀, pH and nitrogen fixing activity were measured (Fig. 4). The nitrogen fixing activity was measured by an established method for evaluating the reduction activity of acetylene to ethylene. Centrifugation was also performed at 15,000 rpm for 5 minutes, and the ammonia concentration of the obtained culture supernatant was quantified using LaboAssay^{™} ammonia (FujiFilm-Wako Pure Chemical Industries) (Fig. 4). As a result, nitrogen fixing activity increased and the pH also increased in DC#3 strain to which IPTG is added, compared to DC#3 to which IPTG is not added. 400 mg/L of ammonia accumulated at the point where nitrogen fixing activity disappears. PCR was conducted by amplifying a high expression cassette using cell solutions of strain DC#3 before and after main culturing. The primers used are shown in Table 7. As a result, the high expression cassette (6647 bp) which was present before the main culturing was retained even after culturing, with no observable return to the wild type gene fragment (2955 bp) (Fig. 5). This demonstrated that gene fragments introduced into the algU gene locus by double crossover are stably retained in the genome, and that high-concentration ammonia accumulates in the medium.

**[Table 7]**

| Table 7. Primers used to confirm transferred fragment | | |
|---|---|---|
| Primer name | Sequence | Sequence No. |
| F16 | GGCGAGTAGAGTCTTGAAGATGG | 31 |
| R16 | TGTTGACGAAGATAGGTGGGAAGC | 32 |

### Example 5: Confirmation of ammonia accumulation stability by DC#3

In order to confirm the ammonia accumulation stability of cryopreserved DC#3, a glycerol stock was prepared and cryopreserved at -80°C, after which the stock was thawed and used for culturing of DC#3. Preculturing (30°C, 280 rpm) of DC#3 was carried out in a test tube containing 5 mL of Burk's+N medium with addition of kanamycin at a final concentration of 1 µg/mL. It was then inoculated into a test tube containing 5 mL of Burk's medium to an OD₆₀₀ = 0.02, and shake culturing was carried out (30°C, 280 rpm). IPTG was added to a final concentration of 0.0 3 mM 20 hours after the start of culturing. The culture solution was collected at 70 to 80 hours after the start of culturing, and after centrifugation at 15,000 rpm for 5 minutes, the pH and ammonia concentration of the obtained culture supernatant were quantified using LaboAssay^{™} ammonia (FujiFilm-Wako Pure Chemical Industries) (Table 8). The main culture test was carried out independently three times, using strains frozen and thawed once for Lot1, and strains frozen and thawed twice for Lots 2 and 3. The three independent experiments showed that DC#3 stably accumulates about 600 mg/L of ammonia. These results demonstrated that DC#3 stably accumulates ammonia even with repeated freezing and thawing of cryopreserved stock.

**[Table 8]**

| Table 8. Confirmation of ammonia production by frozen stocks | | | | |
|---|---|---|---|---|
| | | Lot 1 | Lot 2 | Lot 3 |
| Wild type | pH | 6.6 | 6.9 | 6.8 |
| | Ammonia concentration (mg/L) | 0.0026 | 2.1 | 1.6 |
| DC#3 | pH | 9 | 9 | 8.9 |
| | Ammonia concentration (mg/L) | 614 | 585 | 674 |

### Example 6: Culturing of strain DC#3 in 500 mL-volume Erlenmeyer flask

The frozen DC#3 glycerol stock was thawed and DC#3 was cultured. Preculturing (30°C, 280 rpm) of DC#3 was carried out in a test tube containing 5 mL of Burk's+N medium with addition of kanamycin at a final concentration of 1 µg/mL. It was then inoculated into a baffled Erlenmeyer flask containing 100 mL of Burk's medium comprising 2% sucrose or ethanol as a carbon source, to OD₆₀₀ = 0.02, and shake culturing was carried out (30°C, 100 rpm). *IPTG was added at a final concentration of 0.0 3 mM for* the culture medium comprising sucrose as the carbon source at 20 hours after the start of culturing, and for the culture medium comprising sucrose as the carbon source at 30 hours after the start of culturing.
. The culture solution was collected at 72 hours after the start of culturing, and after centrifugation at 15,000 rpm for 5 minutes, the pH and ammonia concentration of the obtained culture supernatant were quantified using LaboAssay^{™} ammonia (FujiFilm-Wako Pure Chemical Industries) (Table 9). The main culture test was conducted with two independent strains, and the average value was calculated. As a result, it was found that strain DC#3 stores at least 1 g/L of ammonia even in medium comprising sucrose and ethanol as carbon sources. These results demonstrated that DC#3 accumulates a high-concentration of ammonia in a more stable manner than hitherto reported ammonia accumulating strains.

**[Table 9]**

| Table 9. Confirmation of ammonia production in 500 mL Erlenmeyer flask | | | |
|---|---|---|---|
| | Wild type | DC#3 | |
| Medium | Burk's medium w/ 2% sucrose | Burk's medium w/ 2% sucrose | Burk's medium w/ 2% ethanol |
| pH | 6.6 | 8.9 | 8.5 |
| Ammonia concentration (mg/L) | 0.16 | 1040 | 1064 |

### Example 7: Culturing of ammonia-accumulated culture product after culturing of DC#3, and culturing of ammonia-utilizing microorganism

### (Preparation of ammonia-accumulated culture product after DC#3 culturing)

The *Azotobacter vinelandii* DJ wild type and DC#3 were inoculated into a 500 mL-volume baffled Erlenmeyer flask containing 100 mL of Burk's medium, and shake culturing was carried out at 30°C, 100 rpm. After centrifuging the culture solution at 7,000 rpm for 10 minutes starting from 72 hours after the start of culturing, a 0.2 µm filter was used for sterilization to prepare an ammonia-accumulated culture product.

### (Culturing of Bacillus subtilis)

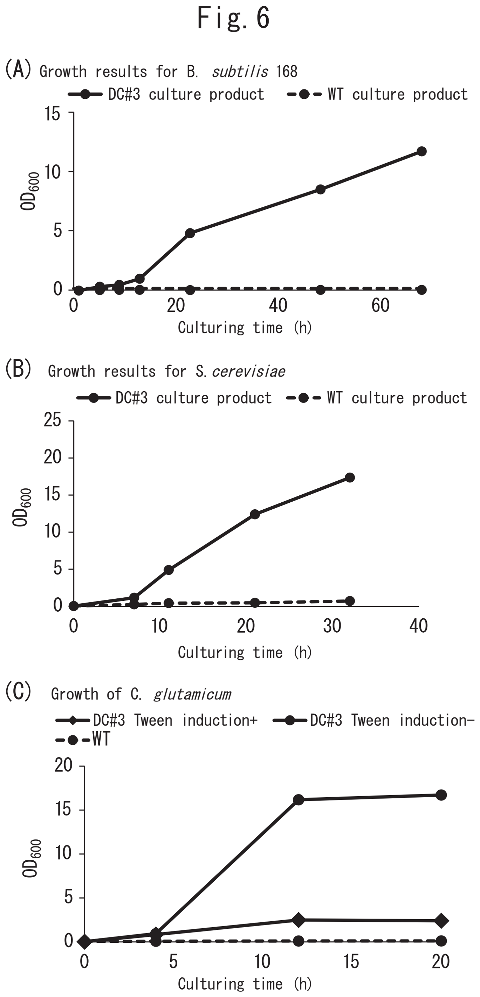

*Bacillus subtilis* 168 was inoculated into a test tube containing 5 mL of LB medium, and shake culturing was carried out overnight at 30°C. After centrifuging the culture solution at 7,000 rpm for 10 minutes, the cells were washed twice with a sterilizing 0.85% (w/v) NaCl solution. The bacterial cell solutions were inoculated into culture solutions of the aforementioned wild type and DC#3, with OD₆₀₀ = 0.02. The following were also added: glucose as a sugar source, to a final concentration of 10 g/L, Yeast Nitrogen Base w/o Amino Acids and Ammonium Sulfate (Becton, Dickinson and Company) as trace elements to a final concentration of 1.7 g/L, and 50 µg/mL tryptophan as an essential requirement. Shake culture was carried out at 30°C, and the time-dependent change in OD₆₀₀ was measured (Fig. 6A). As a result, growth did not occur in the wild type culture solution whereas growth was confirmed in the DC#3 culture solution.

### (Culturing of Saccharomyces cerevisiae)

*Saccharomyces cerevisiae* KY23 was inoculated into a test tube containing 5 mL of YPD medium, and shake culturing was carried out overnight at 30°C. After centrifuging the culture solution at 7,000 rpm for 10 minutes, the cells were washed twice with a sterilizing 0.85% (w/v) NaCl solution. The OD₆₀₀ of each cell solution was measured, and the solution was inoculated into culture solutions of the aforementioned wild type and DC#3, to OD₆₀₀ = 0.02. The following were added: glucose as a sugar source, to a final concentration of 20 g/L, and Yeast Nitrogen Base w/o Amino Acids and Ammonium Sulfate (Becton, Dickinson and Company) as trace elements, to a final concentration of 1.7 g/L. Shake culture was carried out at 30°C, and the time-dependent change in OD₆₀₀ was measured (Fig. 6B). As a result, growth did not occur in the wild type culture solution whereas growth was confirmed in the DC#3 culture solution.

### (Culturing and glutamic acid production with Corynebacterium glutamicum)

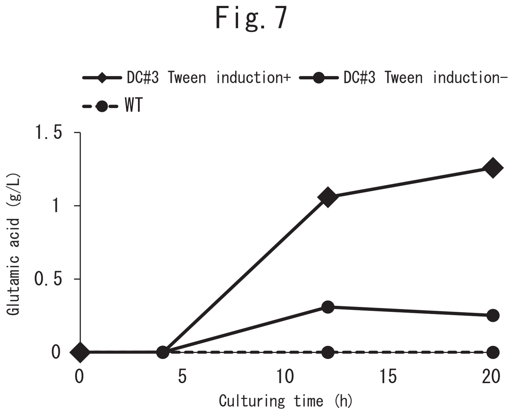

*Corynebacterium glutamicum* ATCC13032 was inoculated into a test tube containing 5 mL of LB medium, and shake culturing was carried out overnight at 30°C. After centrifuging the culture solution at 7,000 rpm for 10 minutes, the cells were washed twice with a sterilizing 0.85% (w/v) NaCl solution. The OD₆₀₀ of each cell solution was measured, and the solution was inoculated into culture solutions of the aforementioned wild type and DC#3, to OD₆₀₀ = 0.02. The following were added: glucose as a sugar source, to a final concentration of 10 g/L, and Yeast Nitrogen Base w/o Amino Acids and Ammonium Sulfate (Becton, Dickinson and Company) as trace elements, to a final concentration of 1.7 g/L. In order to induce glutamic acid production, Tween^{R} 40 was added to the medium 4 hours after the start of culturing, to a final concentration of 1.5 g/L. Shake culture was carried out at 30°C, and the time-dependent change in OD₆₀₀ was measured (Fig. 6C). At 20 hours after the start of culturing, the glutamic acid concentration in the culture supernatant was quantified using a "Yamasa" NEO L-glutamic acid measuring kit (Yamasa Corp.) (Fig. 7). As a result, growth did not occur in the wild type culture solution whereas growth was confirmed in the DC#3 culture solution. Moreover, while glutamic acid was not produced in the wild type culture solution, inducing production with Tween^{R} in the DC#3 culture solution resulted in production of approximately 1.26 (g/L) glutamic acid. These results demonstrated that it is possible to grow food microorganisms and produce glutamic acid using only a nitrogen source obtained by fixing nitrogen in the air.

### Example 8: Use of cultured ammonia-utilizing microorganisms for food production

### (Production of meat alternative)

The ammonia-accumulated culture product obtained from culturing the DC#3 prepared in Example 7, and *Aspergillus oryzae,* were inoculated into 300 mL of the ammonia-accumulated culture product obtained from culturing of DC#3. The following were added: glucose as a sugar source, to a final concentration of 10 g/L, and Yeast Nitrogen Base w/o Amino Acids and Ammonium Sulfate (Becton, Dickinson and Company) as trace elements, to a final concentration of 1.7 g/L. The pH was adjusted to 6.5 and shake culturing was carried out at 30°C. The culture solution was filtered with filter paper (7C by Advantec) to collect the hyphae. The collected hypha were washed 3 times with purified water and the moisture was removed by pressing and suction to obtain the hyphae as a pellet, which was provided as a meat alternative. After mixing 20 g of the viable hyphae pellet, 2 g of palm oil and 1 g of strong flour, the mixture was used to produce a hamburger.

### (Production of soy sauce)

The ammonia-accumulated culture product obtained from culturing of DC#3 prepared in Example 7, and *Saccharomyces cerevisiae,* were inoculated into 300 mL of the ammonia-accumulated culture product obtained from culturing of DC#3. The following were added: glucose as a sugar source, to a final concentration of 20 g/L, and Yeast Nitrogen Base w/o Amino Acids and Ammonium Sulfate (Becton, Dickinson and Company) as trace elements, to a final concentration of 1.7 g/L. Shake culture was carried out at 30°C, and the obtained yeast cells were freeze-dried to obtain dry yeast cells. Steam-denatured defatted soybean, crushed roasted wheat and dried yeast were mixed in a proportion of 1:2:1, and then *Aspergillus sojae* seed koji was inoculated and a common method was used for 42 hours of koji fermentation to obtain soy sauce koji. After charging 100 parts by mass of the obtained soy sauce koji into 130 parts by mass of brine (salt concentration: 26% (w/v)), the mash was monitored and controlled by an ordinary method for 150 days with discretionary stirring at 25 to 30°C, and fermented and aged. The resulting aged mash was pressed and filtered to produce raw soy sauce.

### (Production of miso)

The ammonia-accumulated culture product obtained from culturing of DC#3 prepared in Example 7, and *Saccharomyces cerevisiae,* were inoculated into 300 mL of the ammonia-accumulated culture product obtained from culturing of DC#3. The following were added: glucose as a sugar source, to a final concentration of 20 g/L, and Yeast Nitrogen Base w/o Amino Acids and Ammonium Sulfate (Becton, Dickinson and Company) as trace elements, to a final concentration of 1.7 g/L. Shake culture was carried out at 30°C, and the obtained yeast cells were freeze-dried to obtain dry yeast cells. The steamed-denatured beans and dried yeast were mixed in equal amounts to prepare miso balls, and then kosen (roasted and crushed wheat) and seed koji were dispersed and used for koji making for 42 hours by a common method. Salt water was added to the obtained koji to a salt content of about 11% (w/v), and the mixture was fermented and aged for 6 months at about 30°C by a common method, to produce bean miso.

### INDUSTRIAL APPLICABILITY

The nitrogen-fixing bacteria of the invention grow in nitrogen limited medium and have enhanced nitrogen-fixing ability, and can accumulate ammonia at a higher concentration than the prior art. The cells themselves can therefore be efficiently utilized as a protein source for foods and feeds. Moreover, the ammonia-accumulated culture product may be used not only as fertilizer or fuel, but also for culturing of ammonia-utilizing microorganisms to produce amino acids and/or protein-containing culture products utilizing nitrogen in the atmosphere. Furthermore, application of the nitrogen-fixing bacteria of the invention to a microbial preparation can be expected to reduce the amount of nitrogen fertilizer used. From this viewpoint, the nitrogen-fixing bacteria and ammonia accumulated product of the invention make it possible to avoid the use of nitrogen compounds fixed via high energy consumption, as in the conventional Haber-Bosch method.

[Sequence Listing]

## Claims

1. Nitrogen-fixing bacteria retaining the endogenous nifL-nifA gene locus, and having the nifA gene introduced by homologous recombination into a genomic region other than the nifL-nifA gene locus.

2. Nitrogen-fixing bacteria according to claim 1, wherein a nifA gene expression cassette comprising the nifA gene and an expression regulatory sequence disposed in manner allowing regulation of expression of the nifA gene, is introduced by homologous recombination into a gene locus other than the nifL-nifA gene locus.

3. Nitrogen-fixing bacteria according to claim 1, wherein the nitrogen-fixing bacteria are *Azotobacter* bacteria.

4. Nitrogen-fixing bacteria according to claim 1, wherein the nitrogen-fixing bacteria are *Azotobacter vinelandii* bacteria.

5. Nitrogen-fixing bacteria according to claim 1, wherein the nifA gene expression cassette is introduced at a gene locus other than the nifL-nifA gene locus using the double crossover method.

6. An ammonia-accumulated culture product obtained by culturing nitrogen-fixing bacteria according to any one of claims 1 to 5 in nitrogen limited medium in the absence of a selection agent.

7. The culture product according to claim 6, which contains ammonia at 510 mg/L or greater.

8. A method for producing an ammonia-accumulated culture product, comprising culturing nitrogen-fixing bacteria according to any one of claims 1 to 5 in nitrogen limited medium in the absence of a selection agent.

9. A method for producing an amino acid- or protein-containing culture product, comprising:
a step of obtaining an ammonia-accumulated culture product by the method according to claim 8, and
a step of culturing an ammonia-utilizing microorganism in the ammonia-accumulated culture product to obtain an amino acid- or protein-containing culture product.

10. The method according to claim 9, which comprises a step of culturing nitrogen-fixing bacteria and inducing expression of the transferred nifA gene.

11. The method according to claim 9, wherein the ammonia-accumulated culture product contains a culture supernatant and/or cell homogenate.

12. The method according to claim 9, wherein the amino acid- or protein-containing culture product is used for a food.

13. The method according to claim 9, wherein a sugar is added to the ammonia-accumulated culture product in which the ammonia-utilizing microorganism is to be cultured.

14. The method according to claim 9, wherein the ammonia-utilizing microorganism is selected from the group consisting of microorganisms belonging to *Corynebacterium,* microorganisms belonging to *Bacillus,* microorganisms belonging to *Saccharomyces,* microorganisms belonging to *Aspergillus,* and microorganisms belonging to *Fusarium.*
